# EUROPEAN PATENT SPECIFICATION

(11) **EP 0 975 569 B1**
(45) Date of publication and mention of the grant of the patent: **03.09.2003**
(21) Application number: 97919377.8
(22) Date of filing: 17.04.1997
(51) Int. Cl.: C07C 37/60, C07C 39/30, C07C 65/21, C07C 275/54

(54) **PROCESS FOR THE HYDROXYLATION OF 1,4-DICHLOROBENZENE**
VERFAHREN ZUR HYDROXYLIERUNG VON 1,4-DICHLORBENZOL
PROCEDE SERVANT A HYDROXYLER 1,4-DICHLOROBENZENE

(43) Date of publication of application: 02.02.2000
(73) Proprietor: Syngenta Participations AG, 4058 Basel (CH)
(72) Inventor: SCHEUERMAN, Randall, A., Santa Clara, CA 95050 (US); HENRICK, Clive, A., Palo Alto, CA 94303 (US)
(86) International application number: EP9701921
(87) International publication number: WO98047843

(56) References cited:
- EP-A- 0 179 022
- WO-A-92/18449
- DE-A- 2 101 992
- GB-A- 1 428 271
- US-A- 3 013 054
- US-A- 4 529 824
- K. FUJIMOTO, ET AL.: "Selective one-pot formation of phenols by anodic oxidation" TETRAHEDRON, vol. 52, no. 11, 11 March 1996, OXFORD, GB, pages 3889-3896, XP002049068
- M. BIANCHI, ET AL.: "Hydroxylation of aromatics with hydrogen peroxide catalysed by vanadium(V) peroxo complexes" JOURNAL OF MOLECULAR CATALYSIS, vol. 83, no. 1-2, 1993, AMSTERDAM, NL, pages 107-116, XP002049592 cited in the application
- JERRY MARCH: "Advanced Organic Chemistry", 1992, JOHN WILEY & SONS, NEW YORK
- Chem. Technol. 1973 (3), 120-128, D. M. Jerina, "Hydroxylation of aromatics, chemical models for the biological processes"

## Description

The present invention concerns the selective oxidation of 1,4-dichlorobenzene to 2,5-dichlorophenol.

US Patent 4,529,824 describes in general certain complexes of vanadium, niobium and tantalum and their use in hydroxylating aromatic hydrocarbons either as reactant or catalyst. Additionally, Moiseeva et al. Kinet. Katal 29(4), 970-4 (1988) describe oxidation of benzene with vanadium compounds albeit not selectively for phenol (oxidation proceeds at least in part to quinone).

It has now surprisingly been found that oxidation of 1,4-dichlorobenzene to 2,5-dichlorophenol can be effected with high yield and selectivity when carried out in the presence of certain metal derivatives and in the presence of formic or an alkanoic acid.

The present invention therefore provides a process for the preparation of 2,5-dichlorophenol which comprises selectively oxidizing 1,4-dichlorobenzene using an oxidizing agent and a peroxo-, hydroperoxo-, superoxo- or alkylperoxo-metal species wherein said metal species are free of complex ligands and the metal is selected from scandium, yttrium, lanthanum, titanium, zirconium, hafnium, vanadium, niobium, tantalum, chromium, molybdenum, tungsten, manganese, rhenium, iron, ruthenium, selenium, tellurium, osmium, cobalt, rhodium, iridium, nickel, palladium, platinum, copper, silver, zinc, aluminum, gallium, indium, tin, cerium, praseodymium, neodymium, samarium, europium, gadolinium, terbium, dysprosium, holmium, erbium, thulium, ytterbium, lutetium, and uranium or mixtures thereof in the presence of an acid selected from formic or an alkanoic acid.

The oxidation may be effected using an oxidation agent. Suitable oxidation agents included organic peroxides such as peroxycarboxylic acids, RCO₃H, alkyl hydroperoxides, ROOH, and dialkylperoxides, ROOR, for example peroxyacetic acid, peroxybenzoic acid, peroxyformic acid, t-butyl hydroperoxide, and di-t-butylperoxide and inorganic peroxides such as peroxydisulfuric acid. The preferred peroxide for use in the invention however, is hydrogen peroxide, especially in aqueous solution. The present process can be carried out at dilutions of 3% to 90% with almost complete utilization of H₂O₂ and quantitive yields. However, ca 50% aqueous H₂O₂ is preferred. The proportions of H₂O₂ to 1,4-dichlorobenzene may vary between 0.2 and 5, e.g. between 0.5 and 2. It has been determined that particularly high efficiency may be achieved using approximately equimolar amounts of H₂O₂ and 1,4-dichlorobenzene, e.g. ca 0.9 to ca 1.1 molar proportion of H₂O₂ per mole of 1,4-dichlorobenzene.

The peroxo-, hydroperoxo-, superoxo- or alkylperoxo-metal species may be and preferably is generated in situ by adding the desired metal either as pure metal or in the form of a metal oxide or other suitable form. Alternatively, the desired derivative e.g. an acetoacetonate may be formed prior to reaction and added to the reaction mixture.

Suitable metal forms are, anhydrides, acetylacetonates, alkoxymetal derivatives, sulfates, nitrates, halides, oxyhalides, alkylthiocarbamates or metalates with other cations, (e.g. ammonium metavanadate NH₄VO₃).

Preferred metals include molybdenum, titanium, vanadium, tungsten, rhenium, selenium, niobium, tantalum and tellurium with vanadium being particularly preferred.

Preferred metal forms include oxides, acetylacetonates, alkoxymetal oxides and oxychlorides.

A discussion of peroxo-, hydroperoxo-, superoxo- and alkylperoxo-metal species can be found in Conte et al. in Organic Peroxides Ed. W. Ando, John Wiley & Sons (1992) (pp 559-598).

Discussion of hydroperoxide oxidising agents and metal derivatives may also be found in US Patents 3,350,422; 3,351,635; 3,360,584; 3,360,585; and 3,662,006.

A particular advantage of the present process is that the peroxo-, hydroperoxo-, superoxo- or alkylperoxo-metal species are free of complex ligands.

The metal derivatives can be present in amount equivalent to between 0.001 and 100 mol % of metal. Preferably catalytic amounts of 0.1 to 15 mol % are employed.

When employed as a catalyst the metal derivative may be recycled and regenerated. For example when an oxide such as vanadium pentoxide is used the spent catalyst can be heated in air (cf Polish Patent PL 73-165695; C.A. 87.91418) or treated with hydrogen peroxide.

The acid component of the process according to the invention is selected from formic or an alkanoic acid. Preferred alkanoic acids are lower alkanoic acids containing e.g. 2 to 6 carbon atoms such as acetic, propionic or butyric acid. The preferred acid according to the invention is acetic acid.

The use of solvents may also have a beneficial effect on the process according to the invention. Examples of such solvents include aprotic solvents such as chlorinated solvents e.g. 1,2-dichloroethane, dichloromethane and chloroform, nitrated solvents, e.g. nitromethane; nitriles e.g. acetonitrile, propionitrile and benzonitrile; ketones, e.g. acetone and methylethylketone; alkanes, e.g. hexane; esters e.g. ethylacetate; alcohols e.g. methanol, ethanol and isopropanol; or mixtures thereof.

Reaction temperatures lie between 0 and 150ºC and are preferably in the range of room to elevated (ca 90º) temperature e.g. when employing acetic acid and a vanadium derivative. The reaction is preferably carried out in the substantial absence of water. In certain cases removal of water further increases yield. This is the case for example where the oxidising agent is used in aqueous solution or where the water is generated during the reaction. Removal of water can be achieved for example by carrying out the reaction in the presence of a drying agent such as activated molecular sieves (3Å or 4Å), calcium sulphate, magnesium sulphate or acetic anhydride, etc.

In certain cases pH will affect the course of the reaction and therefore the addition of a Lewis acid or a Brønsted acid (cf Kirk Othmer Encyclopedia of Chemical Technology; 4th Edition; Wiley Interscience) such as p-toluenesulfonic or methanesulfonic add can increase the yield.

Addition of a salt of the chosen formic or alkanoic acid e.g. dry sodium acetate with acetic acid can increase the yield in certain cases.

The reaction mixture may be mono- or multi- e.g. bi-phasic. Examples of such systems can be found in Bianchi et al. J. Mol. Catal. 83 (1993) 107-116; Bonchio et al. J. Org. Chem. 54 (1989) 4368-4371.

Typically the substrate to be oxidised (1,4-dichlorobenzene) is dissolved in the chosen acid (e.g. acetic acid) and the metal derivative (e.g. a high valency oxide) added followed by the slow addition of the oxidising agent (e.g. aqueous H₂O₂). The metal derivative may be deposited on a carrier such as silica, alumina, aluminosilicates, zeolites, coals, titanium oxide, quartz, etc.

The starting material 1,4-dichlorobenzene has the formula 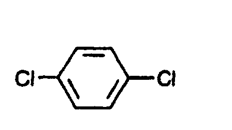 and is a known, commercially available substance.

The desired end product 2,5-dichlorophenol has the formula 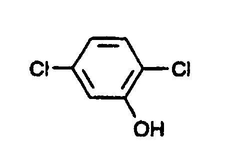 and is useful as an intermediate in the preparation of the commercial herbicide dicamba in high isomeric purity (e.g. in substantial absence of 3,5-dichloro-o-anisic acid).

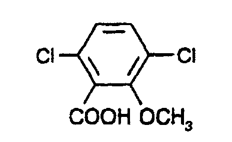

This process involves carboxylation of the 2,5-dichlorophenol to give 2-hydroxy-3,6-dichlorobenzoic acid (a.k.a. 3,6-dichlorosalicylic acid) and methylation of this substrate with subsequent saponification to give high purity dicamba which may be isolated in free acid, salt or ester form (cf e.g. US Patent 3,013,054 for reaction from 2,5-dichlorophenol to dicamba).

In addition, the end product 2,5-dichlorophenol is also useful as an intermediate in the preparation of the commercial acaricide lufenuron. 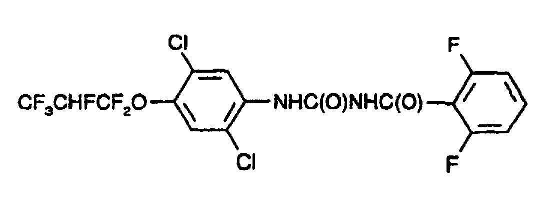 This process involves either (1a) nitration of the 2,5-dichlorophenol to give the 2,5-dichloro-4-nitrophenol and (2a) hydrogenation of the nitro group to give the required intermediate 2,5-dichloro-4-aminophenol, or (1b) haloalkylation of the 2,5-dichlorophenol to give the 2,5-dichloro-haloalkoxybenzene, (2b) nitration of the benzene ring to give the 2,5-dichloro-4-nitro-haloalkoxybenzene and (3b) subsequent reduction of the nitro group to give the required intermediate 2,5-dichloro-4-amino-haloalkoxybenzene.
The resulting 2,5-dichloro-4-amino-haloalkoxybenzene is then transferred to the corresponding isocyanate derivative, for example with phosgene, and reacted with halobenzamide, for example 2,6-difluorobenzamide, to give lufenuron.

The preparation of lufenuron from 2,5-dichloro-4-nitrophenol and 2,5-dichloro-4-aminohaloalkoxybenzene respectively is described in EP-A-0 179 021 and EP-A-0 179 022.

The following examples illustrate the invention. Temperatures are in degrees centigrade.

### EXAMPLE 1

To a solution of 1.47 g (10 mmol) of 1,4-dichlorobenzene dissolved in 20 mL of acetic acid is added with stirring 0.182 g (1 mmol) vanadium(V) oxide followed by the gradual infusion of 2mL of 50% aqueous hydrogen peroxide (30 mmol) over 8 hours. After 24 hours at 20ºC the solid Is removed by filtration, and washed with methanol. The combined filtrates are treated dropwise with concentrated aqueous sodium bisulfite to destroy any residual hydrogen peroxide and the solvents are removed in vacuo to give the desired product. This may be purified e.g. by vacuum distillation (b.p. 70ºC at 0.2 mbar pressure) to give 2,5-dichlorophenol, m.p. 57-59ºC.

### EXAMPLE 2

To a solution of 1.47 g (10 mmol) of 1,4-dichlorobenzene dissolved In 20 mL of acetic acid is added with stirring 1.0 g of ∼3.5 weight percent vanadium(V) oxide deposited on silica (0.2 mmol vanadium catalyst) the reaction is heated to 45ºC followed by the gradual infusion of 0.67 mL of 50% aqueous hydrogen peroxide (10 mmol) over 8 hours. After 16 hours at 45ºC the solid is removed by filtration, and washed with methanol. The combined filtrates are treated dropwise with concentrated aqueous sodium bisulfite to destroy any residual hydrogen peroxide and the solvents are removed in vacuo to give the desired product. This may be purified e.g. by vacuum distillation (b.p. 70ºC at 0.2 mbar pressure) to give 2,5-dichlorophenol, m.p. 57-59ºC.

### EXAMPLE 3

To a solution of 1.47 g (10 mmol) of 1,4-dichlorobenzene dissolved In 20 mL of acetic acid is added with stirring 265 mg (1 mmol) vanadyl acetylacetonate followed by the gradual infusion of 2.0 mL of 50% aqueous hydrogen peroxide (30 mmol) over 8 hours. After 16 hours at 20ºC the solid is removed by filtration, and washed with methanol. The combined filtrates are treated dropwise with concentrated aqueous sodium bisulfite to destroy any residual hydrogen peroxide and the solvents are removed in vacuo to give the desired product. This may be purified e.g. by vacuum distillation (b.p. 70ºC at 0.2 mbar pressure) to give 2,5-dichlorophenol, m.p. 57-59ºC.

### Example 4: 2,5-Dichloro-(1,1,2,3,3,3-hexafluoropropoxy)-benzene

Molten 2,5-dichlorophenol (1.0 mol) is dissolved in acetonitrile, potassiumhydroxide (0.2 mol) and water. To the solution, hexafluoropropylene (1.025 mol) is added at 25-30°C over 8 hours. Dilute hydrochloric acid (10% water solution) is added simultaneously to hold the pH-value of the solution at 8.5-9.5. After the reaction is completed (controlled by gaschromatography) 500 ml of acetonitrile/water is distilled off at 60°C/600 mbar. Finally the lower layer is separated and the product is distilled at 80°C and 10 mbar.

### Example 5: 2,5-Dichloro-(1,1,2,3,3,3-hexafluoropropoxy)-4-nitrobenzene

2,5-Dichloro-(1,1,2,3,3,3-hexafluoropropoxy)-benzene (1.0 mol, Example 4) is dissolved in concentrate sulfuric acid. Then a mixture of fuming nitric acid (1.15 mol) and concentrated sulfuric acid (0.75 mol) is added over 3 hours at 25-40°C. To complete the reaction, the mixture is stirred for a further 3 hours (conversion control by gaschromatography). Then the mixture is poured into cold water and neutralized with sodium hydroxide. The mixture is extracted with toluene. Finally the nitro-product is isolated by evaporation of the toluene at 50-60°C and 100-200 mbar.

## Claims

1. A process for the preparation of 2,5-dichlorophenol which comprises selectively oxidizing 1,4-dichlorobenzene using an oxidizing agent and a peroxo-, hydroperoxo-, superoxo- or alkylperoxo-metal species wherein said metal species are free of complex ligands and the metal is selected from scandium, yttrium, lanthanum, titanium, zirconium, hafnium, vanadium, selenium, tellurium, niobium, tantalum, chromium, molybdenum, tungsten, manganese, rhenium, iron, ruthenium, osmium, cobalt, rhodium, iridium, nickel, palladium, platinum, copper, silver, zinc, aluminum, gallium, indium, tin, cerium praseodymium, neodymium, samarium, europium, gadolinium, terbium, dysprosium, holmium, erbium, thulium, ytterbium, lutetium, and uranium or mixtures thereof in the presence of an acid selected from formic or alkanoic acids.

2. A process according to Claim 1 wherein the metal is selected from molybdenum, titanium, vanadium, tungsten, rhenium, selenium, niobium, tantalum and tellurium.

3. A process according to Claim 1 wherein the metal is vanadium.

4. A process according to Claim 1 wherein the oxidizing agent is a peroxide.

5. A process according to Claim 1 wherein th oxidizing agent is selected from a peroxycarboxylic acid, an alkylhydroperoxide, a dialkylperoxide.

6. A process according to Claim 1 wherein the oxidizing agent is hydrogen peroxide.

7. A process according to Claim 1 wherein the acid is formic or a C₂ - C₆ alkanoic acid.

8. A process according to Claim 1 wherein the acid is acetic acid.

9. A process according to Claim 1 wherein the peroxo-, hydroperoxo-, superoxo- or alkylperoxo-metal species is generated in situ by adding pure metal or metal-oxide, anhydrides, acetylacetonates, alkoxymetal derivatives, sulfates, nitrates, halides, oxyhalides, alkylthiocarbamates or metalates with other cations.

10. A process according to Claim 9 wherein the metal is added in the form of an oxide, anhydride, acetylacetonate, alkoxymetal derivative, sulfate, nitrate, halide, oxyhalide, alkylthiocarbamate or metalate with other cations.

11. A process according to Claim 10 wherein the metal form is an oxide, acetyl-acetonate, alkoxymetal oxide or oxychloride.

12. A process according to Claim 1 wherein a Lewis acid or a Bronsted acid is additionally present.

13. A process according to Claim 1 wherein a salt of the chosen formic or alkanoic acid is additionally present.

14. A process according to Claim 1 wherein aqueous hydrogen peroxide at a dilution of 50% is used as the oxidizing agent

15. A process according to claim 1 further comprising the carboxylation of 2,5-dichlorophenol to give 2-hydroxy-3,6-dichlorobenzoic acid, methylation of this substrate and saponification of the product to give isomerically pure 3,6-dichloro-o-anisic acid (dicamba) which may be isolated in free acid or salt or ester form.

16. A process according to claim 1 further comprising haloalkylation of the 2,5-dichlorophenol to give the 2,5-dichloro-haloalkoxybenzene, nitration of this substrate to give the 2,5-dichloro-4-nitro-haloalkoxybenzene and subsequent reduction of the nitro group to give the 2,5-dichloro-4-aminohaloalkoxybenzene, followed by transformation of the 4-amino group to the 4-isocyanate group and its reaction with halobenzamide to give (RS)-1-(2,5-dichloro-4-(1,1,2,3,3,3-hexafluoropropoxy)phenyl)-3-(2,6-difluorobenzoyl)urea (lufenuron).

## Patentansprüche

1. Verfahren zur Herstellung von 2,5-Dichlorphenol, umfassend die selektive Oxidation von 1,4-Dichlorbenzen unter Verwendung eines Oxidationsmittels und einer Peroxo-, Hydroperoxo-, Hyperoxo- oder Alkylperoxo-Metallspezies, worin die Metallspezies frei von Komplexliganden sind und das Metall ausgewählt ist aus Scandium, Yttrium, Lanthan, Titan, Zirkonium, Hafnium, Vanadium, Selen, Tellur, Niob, Tantal, Chrom, Molybdän, Wolfram, Mangan, Rhenium, Eisen, Ruthenium, Osmium, Kobalt, Rhodium, Iridium, Nickel, Palladium, Platin, Kupfer, Silber, Zink, Aluminium, Gallium, Indium, Zinn, Cer, Praseodym, Neodym, Samarium, Europium, Gadolinium, Terbium, Dysprosium, Holmium, Erbium, Thulium, Ytterbium, Lutetium und Uran oder Gemischen hiervon, in Gegenwart einer Säure, die aus Ameisen- oder Alkansäuren ausgewählt ist.

2. Verfahren nach Anspruch 1, wobei das Metall aus Molybdän, Titan, Vanadium, Wolfram, Rhenium, Selen, Niob, Tantal und Tellur ausgewählt wird.

3. Verfahren nach Anspruch 1, wobei das Metall Vanadium ist.

4. Verfahren nach Anspruch 1, wobei das Oxidationsmittel ein Peroxid ist.

5. Verfahren nach Anspruch 1, wobei das Oxidationsmittel aus einer Peroxycarbonsäure, einem Alkylhydroperoxid, einem Dialkylperoxid ausgewählt wird.

6. Verfahren nach Anspruch 1, wobei das Oxidationsmittel Wasserstoffperoxid ist.

7. Verfahren nach Anspruch 1, wobei die Säure Ameisen- oder eine C₂ bis C₆-Alkansäure ist.

8. Verfahren nach Anspruch 1, wobei die Säure Essigsäure ist.

9. Verfahren nach Anspruch 1, wobei die Peroxo-, Hydroperoxo-, Hyperoxo- oder Alkylperoxo-Metallspezies in situ durch die Zugabe reinen Metalls oder Metalloxids, Anhydriden, Acetylacetonaten, Alkoxymetallderivaten, Sulfaten, Nitraten, Halogeniden, Oxyhalogeniden, Alkylthiocarbamaten oder Metallaten mit anderen Kationen, erzeugt wird.

10. Verfahren nach Anspruch 9, wobei das Metall in Form eines Oxids, Anhydrids, Acetylacetonats, Alkoxymetallderivats, Sulfats, Nitrats, Halogenids, Oxyhalogenids, Alkylthiocarbamats oder Metallats mit anderen Kationen zugegeben wird.

11. Verfahren nach Anspruch 10, wobei die Metallform ein Oxid, Acetylacetonat, Alkoxymetalloxid oder Oxychlorid ist.

12. Verfahren nach Anspruch 1, wobei zusätzlich eine Lewis-Säure oder eine Brönsted-Säure vorliegt.

13. Verfahren nach Anspruch 1, wobei zusätzlich ein Salz der ausgewählten Ameisen- oder Alkansäure vorliegt.

14. Verfahren nach Anspruch 1, wobei wässeriges Wasserstoffperoxid bei einer Verdünnung von 50 % als das Oxidationsmittel verwendet wird.

15. Verfahren nach Anspruch 1, das weiterhin die Carboxylierung von 2,5-Dichlorphenol, um 2-Hydroxy-3,6-dichlorbenzoesäure zu erhalten, die Methylierung dieses Substrats und die Verseifung des Produkts, um isomer reine 3,6-Dichlor-o-Anissäure (Dicamba) zu erhalten, die in Form einer freien Säure, eines Salzes oder eines Esters isoliert werden kann, umfaßt.

16. Verfahren nach Anspruch 1, das weiterhin die Halogenalkylierung des 2,5-Dichlorphenols, um das 2,5-Dichlor-halogenalkoxybenzen zu erhalten, die Nitrierung dieses Substrats, um das 2,5-Dichlor-4-nitro-halogenalkoxybenzen zu erhalten, und die anschließende Reduzierung der Nitrogruppe, um das 2,5-Dichlor-4-amino-halogenalkoxybenzen zu erhalten, gefolgt von der Umwandlung der 4-Aminogruppe in die 4-Isocyanatgruppe und deren Umsetzung mit Halogenbenzamid, um (RS)-1-(2,5-Dichlor-4-(1,1,2,3,3,3-hexafluorpropoxy)phenyl)-3-(2,6-difluorbenzoyl)harnstoff (Lufenuron) zu erhalten, umfaßt.

## Revendications

1. Procédé de préparation de 2,5-dichlorophénol qui comprend l'oxydation sélective de 1,4-dichlorobenzène au moyen d'un agent oxydant et d'une espèce peroxo-, hydroperoxo-, superoxo- ou alkylperoxo-métallique dans lequel lesdites espèces métalliques sont dépourvues de ligands complexes et le métal est choisi parmi le scandium, l'yttrium, le lanthane, le titane, le zirconium, l'hafnium, le vanadium, le sélénium, le tellure, le niobium, le tantale, le chrome, le molybdène, le tungstène, le manganèse, le rhénium, le fer, le ruthénium, l'osmium, le cobalt, le rhodium, l'iridium, le nickel, le palladium, le platine, le cuivre, l'argent, le zinc, l'aluminium, le gallium, l'indium, l'étain, le cérium, le praséodyme, le néodyme, le samarium, l'europium, le gadolinium, le terbium, le dysprosium, l'holmium, l'erbium, le thulium, l'ytterbium, le lutétium et l'uranium, ou des mélanges de ceux-ci, en présence d'un acide choisi parmi l'acide formique ou les acides alcanoïques.

2. Procédé selon la revendication 1 dans lequel le métal est choisi parmi le molybdène, le titane, le vanadium, le tungstène, le rhénium, le sélénium, le niobium, le tantale et le tellure.

3. Procédé selon la revendication 1 dans lequel le métal est le vanadium.

4. Procédé selon la revendication 1 dans lequel l'agent oxydant est un peroxyde.

5. Procédé selon la revendication 1 dans lequel l'agent oxydant est choisi parmi un acide peroxycarboxylique, un hydroperoxyde d'alkyle, un peroxyde de dialkyle.

6. Procédé selon la revendication 1 dans lequel l'agent oxydant est le peroxyde d'hydrogène.

7. Procédé selon la revendication 1 dans lequel l'acide est l'acide formique ou un acide alcanoïque en C₂-C₆.

8. Procédé selon la revendication 1 dans lequel l'acide est l'acide acétique.

9. Procédé selon la revendication 1 dans lequel l'espèce peroxo-, hydroperoxo-, superoxo- ou alkylperoxo-métallique est produite in situ par addition de métal pur ou d'oxyde métallique, d'anhydrides, d'acétylacétonates, de dérivés alcoxymétalliques, de sulfates, de nitrates, d'halogénures, d'oxyhalogénures, d'alkylthiocarbamates ou de métallates avec d'autres cations.

10. Procédé selon la revendication 9 dans lequel le métal est ajouté sous la forme d'un oxyde, d'un anhydride, d'un acétylacétonate, d'un dérivé alcoxymétallique, d'un sulfate, d'un nitrate, d'un halogénure, d'un oxyhalogénure, d'un alkylthiocarbamate ou d'un métallate avec d'autres cations.

11. Procédé selon la revendication 10 dans lequel la forme métallique est un oxyde, un acétylacétonate, un oxyde alcoxymétallique ou un oxychlorure.

12. Procédé selon la revendication 1 dans lequel un acide de Lewis ou un acide de Brønsted est présent en plus.

13. Procédé selon la revendication 1 dans lequel un sel de l'acide formique ou de l'acide alcanoïque choisi est présent en plus.

14. Procédé selon la revendication 1 dans lequel du peroxyde d'hydrogène aqueux à une dilution de 50% est utilisé comme agent oxydant.

15. Procédé selon la revendication 1 comprenant, en outre, la carboxylation du 2,5-dichlorophénol pour donner l'acide 2-hydroxy-3,6-dichlorobenzoïque, la méthylation de ce substrat et la saponification du produit pour donner l'acide 3,6-dichloro-o-anisique comme isomère pur (dicamba) qui peut être isolé sous forme d'acide libre ou de sel ou d'ester.

16. Procédé selon la revendication 1 comprenant, en outre, l'halogénoalkylation du 2,5-dichlorophénol pour donner le 2,5-dichlorohalogénoalcoxybenzène, la nitration de ce substrat pour donner le 2,5-dichloro-4-nitrohalogénoalcoxybenzène, puis la réduction du groupe nitro pour donner le 2,5-dichloro-4-aminohalogénoalcoxybenzène, suivie de la transformation du groupe 4-amino en groupe 4-isocyanate et de sa réaction avec un halogénobenzamide pour donner la (RS)-1-(2,5-dichloro-4-(1,1,2,3,3,3-hexafluoropropoxy)phényl)-3-(2,6-difluorobenzoyl)urée (lufénuron).
